# EUROPEAN PATENT SPECIFICATION

(11) **EP 2 519 225 B1**
(45) Date of publication and mention of the grant of the patent: **25.03.2015**
(21) Application number: 09801365.9
(22) Date of filing: 30.12.2009
(51) Int. Cl.: A61K 9/00, A61K 9/70, A61K 31/465

(54) **MELT EXTRUDED NICOTINE THIN STRIPS**
SCHMELZEXTRUDIERTE DÜNNE NIKOTINSTREIFEN
BANDES MINCES DE NICOTINE EXTRUDÉES À L'ÉTAT FONDU

(43) Date of publication of application: 07.11.2012
(73) Proprietor: Novartis AG, 4056 Basel (CH)
(72) Inventor: BRUCE, Caroline, Austin Texas 78705 (US); MANNING, Mark, Lincoln Nebraska 68506 (US)
(74) Representative: Liphardt, Bernd
(86) International application number: PCT/US2009/069823
(87) International publication number: WO 2011/081628

(56) References cited:
- WO-A1-2009/048522
- WO-A2-2007/095600
- US-A- 6 072 100
- "The Merck Index", 2001, Merck Research Laboratories, Whitehouse Station, NJ ISBN: 0911910131 vol. 13, page 6552,
- "Maltitol", European Pharmacopoeia, 1 January 2005 (2005-01-01), pages 1968-1969, XP055016608, Retrieved from the Internet: URL:http://lib.njutcm.edu.cn/yaodian/ep/EP 5.0/16_monographs/monographs_l-p/Maltitol. pdf [retrieved on 2012-01-16]

## Description

### Background of the Invention

This application relates to melt extruded nicotine-containing thin strips that provide sustained release of nicotine for buccal absorption through the oral mucosa.

Edible films or films that dissolve in the mouth have been used in a variety of applications, including drug and vitamin delivery and delivery of breath freshener. (See, for example, US Patents Nos. 5,948,430,6,596,298 and U.S. Pat. No. 6,923,981.) Such films are commonly made by a wet casting process (see, US Patent No. U.S. Pat. No. 7,425,292) but it has also been suggested that extrusion techniques may be employed. (see U.S. Patents. Nos. RE33,093; 6,072,100 and. 6,375,963.) The selection of materials to be used in a thin strip as well as the most suitable manufacturing approach for the thin strip are dependent on the active pharmaceutical ingredient (API) to be included in the strip and the requirements for its delivery. For example, where the API is intended to be delivered in the stomach or intestine, rapid dissolution and passage through the mouth are desired. Conversely, in cases where the delivery desired is a transmucosal delivery in the mouth (for example transbuccal), a much slower dissolution time is desired. In addition, the strip must have the ability to carry (and then release) a sufficient amount of the API, and the API must not be damaged or destroyed in the manufacturing process.

Various products are known to provide nicotine to a person as a replacement for nicotine in cigarettes as part of a smoking cessation program. These include nicotine-containing transdermal patches (see US Patent No. RE37,934), nasal sprays (see US Patent No. 6,596,740) and nicotine lozenges (see US Patent No. 4,967,773) and chewing gums (see US Patent No. 6,344,222).

US Patent Publication No. 2009/0098192 discusses many of the challenges associated with thin strip formulations, and discloses an extruded thin strip composition. Specific examples were made with hydroxypropylcellulose (HPC) containing APIs including tobacco or snuff. No information about the dissolution time of these strips is provided in the publication. US Patent No. 4,764,378, discloses buccal dosage forms for transmucosal administration of drugs including nicotine that comprise a pharmaceutical compound dispersed in an erodible matrix comprising from about 20 to about 75 percent by weight of a low molecular weight polyethylene glycol component, from about 2 to about 65 percent by weight of a medium or high molecular weight polyethylene glycol component, from about 1% to about 40% percent by weight of an auxiliary high molecular weight polymer. Preferred auxiliary high molecular weight polymers are polyethylene oxide and polyvinylpyrrolidone which improve the molding properties of the matrix and provide water-activated adhesive properties to the compositions to provide a buccal dosage form.

US Patent Publication No. 2008/0260807 discloses a water-soluble film comprising nicotine in which a nicotine-containing fluid is applied to at least one surface of a preformed water-soluble film.

### Summary of the Invention

The present invention provides melt extrusion methods of forming thin strips and thin strips that dissolve in the mouth for delivering nicotine through the oral mucosa over a period of time suitable for supporting smoking cessation. In accordance with the invention, the thin strip is between 0.05 millimeters and 2.00 millimeters thick and comprises:
10 to 80 % by weight of polyethylene oxide having a molecular weight of from 70,000 to 300,000 daltons;
5 to 50 % by weight of a sugar alcohol having a melting point in excess of 75 °C;
5 to 30 % by weight of polyethylene glycol having a molecular weight of from 100 to 4,000 daltons;
1 to 30 % by weight of a carboxy vinyl polymer cross linked with an allyl ether of pentaerythritol; and
1 to 10 % by weight of an organic acid addition salt of nicotine.

Each strip preferably contains from 1 to 8 mg of nicotine (free base equivalent), for example 2 or 4 mg of nicotine. The thin strips preferably have dimension that are suitable for oral ingestion by a user (e.g. 22mm x 16 mm to 22mm x 37 mm). In another embodiment the test strip is formed such that the total surface area of the strip is between 1 cm² to 20 cm² (e.g. between 1 cm² and 10 cm²).

The thin strip is formed by a melt extrusion process. The method includes the steps of: (I) forming a composition comprising thin strip components; (II) hot melt extruding a thin sheet having to a thickness of between 0.05 millimeters and 2.00 millimeters from the composition; and (III) cutting the thin sheet into thin strips. The processing temperature during steps (I), (II), and (III) does not exceed the melting point temperature of the sugar alcohol.

In another embodiment, the present invention provides a thin strip comprising:
10 to 80 % by weight of polyethylene oxide having a molecular weight of from 70,000 to 300,000 daltons;
5 to 50 % by weight of a sugar alcohol having a melting point in excess of 75 °C;
5 to 30 % by weight of polyethylene glycol having a molecular weight of from 100 to 4,000 daltons;
1 to 30 % by weight of a carboxy vinyl polymer selected from the group consisting of a carboxy vinyl polymer cross linked with divinyl glycol or a carboxy vinyl polymer cross linked with an allyl ether of pentaerythritol; and
1 to 10 % by weight of an active pharmaceutical ingredient adapted for transmucosal absorption into the blood stream, wherein the thin strip is between 0.05 millimeters and 2.00 millimeters thick.

### Brief Description of the Drawings

Figure 1 shows the effect of formulation and film thickness on film disintegration time (Examples 1-4).
Figure 2 shows the effect of isothermal hold on weight of nicotine bitartrate dihydrate.
Figure 3 shows the correlation of film thickness and film weight of melt extruded films containing Nicotine bitartrate dihydrate.
Figure 4 shows the disintegration time of melt extruded films containing nicotine bitartrate dihydrate (n=3).
Figure 5 shows the correlation of film thickness and disintegration time of melt extruded films containing nicotine bitartrate dihydrate (Examples 5-9).
Figure 6 shows the correlation of film weight and disintegration time of melt extruded films containing nicotine bitartrate dihydrate (Examples 5-9).
Figure 7 shows the potency of melt extruded films containing nicotine bitartrate dihydrate as determined by the original method.
Figure 8 shows the dose of Nicotine bitartrate dihydrate in melt extruded films.
Figure 9 shows the correlation of film weight and dose of nicotine bitartrate dihydrate in melt extruded films (N=6).
Figure 10 shows the correlation of film thickness and dose of nicotine bitartrate dihydrate in melt extruded films (N=6).

### Detailed Description of the Invention

Numerical values in the specification and claims of this application, particularly as they relate to polymeric materials, reflect average values for a composition that may contain individual polymer molecules of different characteristics. Furthermore, the numerical values should be understood to include numerical values which are the same when reduced to the same number of significant figures and numerical values which differ from the stated value by less than the experimental error of the measurement technique used to determine the value

Reference throughout the specification to "one embodiment," "another embodiment," "an embodiment," "some embodiments," and so forth, means that a particular element (e.g., feature, structure, property, and/or characteristic) described in connection with the embodiment is included in at least one embodiment described herein, and may or may not be present in other embodiments. In addition, it is to be understood that the described element(s) may be combined in any suitable manner in the various embodiments.

In order to provide an acceptable thin strip formulation for nicotine, a strip formulation desirably has several important characteristics, namely:
(1) the ability to carry sufficient nicotine to provide a desired dose in a strip of a size considered acceptable to a user. Strips that have too little carrying capacity require too large a strip, or the use of too many strips to be considered acceptable by the consumer.
(2) a dissolution time in the mouth that is appropriate to the delivery of nicotine the oral mucosa. Too short a dissolution time results in most of the nicotine being swallowed, while too long a dissolution time results in too slow an absorption to achieve blood levels that are effective to reduce nicotine cravings. Preferably the strip is bioadhesive to further control disintegration time in the mouth.
(3) the capability of being formed into a thin strip without substantial degradation and/or loss of the nicotine in the original formulation.
(4) the ability to prevent degradation and loss of the nicotine over time. The thin strip should have a suitable shelf life so that it can be manufactured, transported, and sold to a consumer while maintaining the desirable properties described herein.

Thin strips can be formed by solvent casting techniques where strip ingredients including an active pharmaceutical ingredient (API) are dissolved or suspended in a carrier solvent. The slurry or solution is then applied to a sheet, or some other surface, having a large surface area where the solvent is driven off from the solution leaving the desired ingredients in thin film form. The solvent casting process is run in a batch mode and requires several pieces of processing equipment including those that deal with solvent recapture and purification. This approach has been found not to be particularly suitable for forming thin strips containing an organic acid addition salt of nicotine. In this regard it has been found that thin strips formed by the solvent casting approach are often too thin to contain desired loading of the organic acid addition salt of nicotine. Furthermore the nicotine salt is very volatile and can decompose in water or solvent. During the expensive and energy-intensive "drying" phase of the solvent casting approach nicotine salt may also be removed with the solvent, thereby also decreasing the effective dosage concentration within the thin strip during formation. The nicotine salt may also transform during the process to form a volatile nicotine base. Furthermore, where a bioadhesive polymer is desired in the thin strip, the solvent casting approach is not acceptable as the polymer can absorb and retain too much moisture to support adequate down stream processing of the solvent cast sheets into strips.

Thin strips can also be formed by a hot melt extrusion process whereby ingredients are combined in, or prior to introduction to, an extruder which heats and mixes the ingredients and melt extrudes a laminar composition which is then calendered and cut/punched to provide thin strips of desired thickness. While a hot melt extrusion process can be run in continuous or semi-continuous modes, with or without water, prior melt extrusion processes and extrusion formulations have been found not particularly suitable for producing acceptable thin strips containing nicotine salt. In particular, throughout development of processes and formulations suitable for producing nicotine salt containing thin strips, the present Inventors found that process parameters including extruder operating temperature, shear, pressure, screw speed, and flow rate *inter alia* can lead to loss of the nicotine salt. The Inventors also found to their surprise that compositions they initially believed to be suitable for melt extruding into acceptable thin strips were in fact not compatible with extrusion processes and/or exhibited undesirable properties when in film form.

### A Hot Melt Extrusion Composition Containing a Nicotine Salt:

In one embodiment, the present invention provides a nicotine salt containing composition suitable for melt extrusion to produce thin strips. The composition allows for the formulation of thin strips that achieve the properties described above. In particular thin strips made from the present composition have the ability to carry a sufficient amount of an organic acid addition salt of nicotine to provide a desired dose nicotine in a strip of a size considered acceptable to a user. The composition comprises polyethylene oxide; a sugar alcohol having a melting point in excess of 75 °C (e.g. mannitol mp=167 °C); low molecular weight polyethylene glycol or a similar plasticizer; a carboxy vinyl polymer cross linked with an allyl ether of pentaerythritol; and an organic acid addition salt of nicotine.

Polyethylene oxide (PEO) suitable for use in the compositions of the present invention has a molecular weight of from 70,000 to 300,000 daltons, for example 100,000 to 200,000 daltons (e.g. preferably about 100,000 daltons). Significantly higher molecular weights, or compositions that include coagulants that cause an increase in molecular weight of the polyethylene oxide are generally not desired. PEO with these characteristics is available from Dow Chemical as POLYOX™ WSR N-10 (Mw about 100,000 Daltons) and POLYOX™ WSR N-80 (Mw about 200,000 Daltons). Of these, POLYOX™ WSR N-10 is frequently preferred.

The PEO is suitably present in the composition of the invention in an amount of 10 to 80 weight %, for 25 to 55 weight % (e.g. 40 weight %). It is noted that PEO is also referred to in the art as polyethylene glycol (PEG). However, since a low molecular weight plasticizer that maybe PEG is also used in the composition, this component is referred to as PEO to maintain a distinction.

The compositions of the invention also include a low molecular weight plasticizer. Such plasticizers include glycerin, propylene glycol, triethyl citrate, and polyethylene glycol (PEG). In a preferred embodiment the low molecular weight plasticizer is PEG, which is miscible with the PEO, having a weight average molecular weight (Mw) of between 100 and 4000 Daltons, more preferably between 300 and 600 Daltons (e.g. 400 Daltons or PEG 400 in liquid form). The PEG is present in an amount of 5 to 30 wt% of the formulation, more preferably between 7 and 15 wt% (e.g. 10 wt%) of the formulation.

The composition of the present invention also contains a water-soluble polyol (e.g. a sugar alcohol). The polyol is selected to have a melting point that is greater than 75 °C, more preferably greater than 90 °C, 100 °C, 110 °C, 130 °C, or greater than 150 °C. The polyol is selected such that its melting point is in excess of the highest temperature at which the formulation will be treated during formation of thin strips. Without intending to be bound by any particular mechanism, it is believed that sugar alcohols are soluble in water and saliva and are effective to control the dissolution rate of the thin strips molded from the composition, with higher levels of sugar alcohol resulting in more rapid dissolution. It is believed that the sugar alcohol dissolves quickly creating a porous matrix in the thin strips for rapid dissolution of the other components. Thus, increased levels of sugar alcohol may be used to offset higher molecular weight PEO. In general, sugar alcohol levels of 5 to 50 weight % of the composition, more preferably between 35 and 45 wt% (e.g. 40 wt%) of the composition.

Specific and non-limiting examples of sugar alcohols useful for this purpose include sorbitol, xylitol, mannitol, lactitol, and maltitol. In other embodiments erythritol may optionally be used as the sugar alcohol or in combination with other sugar alcohols. Of these sorbitol (melting point 95 °C) and mannitol (melting point 167 °C) are particularly preferred, with mannitol being most preferred.

The compositions of the present invention also contain a carboxy vinyl polymer (also known as "acrylic acid polymers") that are cross linked using an allyl ether of pentaerythritol as the cross linker. These polymers are typically polymerized in ethyl acetate and are acceptable in pharmaceutical preparations. They are fluffy and white in appearance and are mildly acidic polymers. They are high molecular weight polymers (e.g. due to heavy crosslinking). "Carbomer" is the non-proprietary name for these materials. Exemplary carbonyl vinyl polymers are carbomer 940, and carbomer 947P, carbomer 974P, carbomer 971P, and carbomer 71G.

Such polymers also go by the name Carbopol polymers and are sold by Lubrizol. A preferred list of acceptable carbonyl vinyl polymers are sold under the tradenames Carbopol 974P, Carbopol 971P, and Carbopol 71G (e.g. Carbopol 971P in granular form). These polymers are cross linked using allyl ethers of pentaerythritol as the cross linker are polymerized in ethyl acetate. These preferred carbomers typically have glass transition temperatures between 100 °C and 110 °C (e.g. 105 °C) without the addition of plasticizers. Of these carbomers Carbopol 974P with a viscosity of 29,400 to 39,400 centi-Poise measured at 25°C and Carbopol 971P with a viscosity of 4,000-11,000 centi-Poise measured at 25°C are sometimes preferred. Carbopol 974P and Carbopol 971P vary in cross-linking density. Carbopol 974P a higher cross-linking density than Carbopol 971P. In general carboxy vinyl polymer is preferably present in an amount corresponding to 1 to 30 wt %, for example 5 to 15 weight % (e.g. 5 weight %) of the composition.

**Information from the manufacturer indicates that Carbopol 974P has the properties in the following table:**

| Test | Specification | Lot Test Frequency¹ | Test Procedure² |
|---|---|---|---|
| Identification | | | |
| Colorimetric test | Pass | 1:200 | USP/NF; Ph. Eur. |
| Gel formation test | Pass | 1:1 | USP/NF; Ph. Eur. |
| Infrared spectrum | Pass | -³ | Ph. Eur.; JPE |
| Precipitate test | Pass | 1:200 | Ph. Eur. |
| Carboxylic Acid Content, Assay % | 56.0-68.0⁴ | 1:1 | USP/NF |
| Viscosity, cP, 25°C | | | |
| Brookfield RVT, 20 rpm, neutralized to pH 7.3 - 7.8 | | | |
| 0.5 wt% mucilage, spindle #6 | 29,400 - 39,400 | 1:1 | USP/NF; Ph. Eur. |
| Loss on Drying, % | 2.0 max | 1:1 | USP/NF |
| Heavy Metals, ppm | | | |
| Total heavy metals, as Pb | 20 max | 1:200 | USP/NF |
| Specific metals: Hg, Pb, As, Sb | 10 max | 1:200 | Lubrizol SA-012 |
| Residual Solvent⁵ | | | |
| Ethyl acetate, % | 0.50 max | 1:1 | Lubrizol SA-009 |
| Benzene, ppm | 0.50 max | 1:1 | Ph. Eur. |
| Residual Monomer, ppm | | | |
| Free acrylic acid | 1,000 max | 1:1 | Lubrizol SA-005 |
| Sulphated Ash, % (Residue on ignition) | 2.5 max | 1:200 | Ph. Eur.; JPE |
| pH, 0.2% Dispersion | 2.5-4.0 | 1:200 | JPE |

**Information from the manufacturer indicates that Carbopol 971P and 71G has the properties in the following table:**

| Test | Specification | Lot Test Frequency¹ | Test Procedure² |
|---|---|---|---|
| Identification | | | |
| Colorimetric test | Pass | 1:200 | USP/NF; Ph. Eur. |
| Gel formation test | Pass | 1:1 | USP/NF; Ph. Eur. |
| Infrared spectrum | Pass | -³ | Ph. Eur.; JPE |
| Precipitate test | Pass | 1:200 | Ph. Eur. |
| Carboxylic Acid Content, Assay % | 56.0-68.0⁴ | 1:1 | USP/NF |
| Viscosity, cP, 25°C | | | |
| Brookfield RVT, 20 rpm, neutralized to pH 7.3 - 7.8 | | | |
| 0.5 wt% mucilage, spindle #5 | 4,000 - 11,000 | 1:1 | USP/NF, Ph. Eur. |
| Loss on Drying, % | 2.0 max | 1:1 | USP/NF |
| Heavy Metals, ppm | | | |
| Total heavy metals, as Pb | 20 max | 1:200 | USP/NF |
| Specific metals: Hg, Pb, As, Sb | 10 max | 1:200 | Lubrizol SA-012 |
| Residual Solvent⁵ | | | |
| Ethyl acetate, % | 0.50 max | 1:1 | Lubrizol SA-009 |
| Benzene, ppm | 0.50 max | 1:1 | Ph. Eur. |
| Residual Monomer, ppm | | | |
| Free acrylic acid | 1,000 max | 1:1 | Lubrizol SA-005 |
| Sulphated Ash, % (Residue on ignition) | 2.5 max | 1:200 | Ph. Eur.; JPE |
| pH, 0.2% Dispersion | 2.5-4.0 | 1:200 | JPE |

The compositions of the present invention also contain 1 to 10 weight %, for example 2 to 6 weight % (e.g. 4.34 weight %) of a nicotine free base in the form of an organic acid addition salt of nicotine. In a preferred embodiment the organic acid addition salt of nicotine is nicotine bitartrate dihydrate.

In some embodiments a basic pH is desirable to facilitate nicotine absorption in the mouth by liberating the free base form of the nicotine bitartrate. In these embodiments it has been found to be preferable that the composition further comprise a buffering agent that imparts an alkaline pH. In most preferred embodiments these buffering agents are selected from the group consisting of: Potassium phosphate monobasic, sodium carbonate, sodium bicarbonate, sodium chloride, sodium phosphate dibasic and sodium phosphate monobasic.

The art of adding other excipients to pharmaceutical preparation is well known in the art and such additions do not depart from the scope of the present invention. For example the compositions of the present invention may be blended with well-known flavoring compositions containing active flavorants to form a flavored blend suitable for hot melt extrusion to form thin strips. A non-limiting list of exemplary active flavorants include capsaicin, pieprine, chavicine, vanillin, vanillyl butyl ether, vanillyl ethyl ether, N-nonanoyl vanillylamide, gingerols, zingerone, and combinations of other natural and artificial flavors such as orange, grape, vanilla, cherry, grape, cranberry, peppermint, spearmint, anise, blueberry raspberry, banana, chocolate, caramel, citrus, strawberry, lemon, and lime. These active flavorants are often blended with a bulk carrier to form a flavoring composition for more efficient and even distribution within the presently contemplated composition. Typically, where a flavoring composition (e.g. an active flavor disposed in a bulk carrier) is used it will make up about 2 to 20 wt% of the thin strip. Other additives (e.g. sweetners and preservatives) are well known in the art and may optionally be blended with the composition.

### A Hot Melt Extrusion Composition Containing a Transmucosal Absorbing API:

In another embodiment the present invention provides another thin strip composition suitable for the transmucosal delivery of active pharmaceutical ingredients. While the thin strip composition outlined above has been found to provide for unexpected prolonged strip disintegration times that are suitable for delivery of an active pharmaceutical ingredient adapted for transmucosal absorption into the blood stream (e.g. nicotine bitartrate), other formulations are contemplated that allow for equal or less prolonged disintegration time. These compositions are suitable for transmucosal (e.g. buccal) absorption and delivery into the blood stream of active pharmaceutical ingredients that are more readily absorbed through the oral mucosa. In the present embodiment, the thin strip is between 0.05 millimeters and 2.00 millimeters thick as described above and comprises,
10 to 80 % by weight of polyethylene oxide having a molecular weight of from 70,000 to 300,000 daltons as described above;
5 to 50 % by weight of a sugar alcohol having a melting point in excess of 75 °C and described above;
5 to 30 % by weight of polyethylene glycol having a molecular weight of from 100 to 4,000 daltons as described above;
1 to 30 % by weight of a carboxy vinyl polymer selected from the group consisting of a carboxy vinyl polymer cross linked with divinyl glycol or a carboxy vinyl polymer cross linked with an allyl ether of pentaerythritol; and
1 to 10 % by weight of an organic addition salt of nicotine.

In the present embodiment, it is noted that the carboxy vinyl polymer may be cross linked with other cross linking agents. The carboxy vinyl polymer is either or both the carboxy vinyl polymer cross linked with an allyl ether ofpentaerythritol as described above and/or a carboxy vinyl polymer cross linked with divinyl glycol. Polymers cross-linked using a divinyl glycol cross linker are also known as polycarbophils and are sold under the tradename Noveon® Polycarbophils. A preferred polycarbophil is Noveon® polycarbophil AA-1 USP. This polymer is typically polymerized and cross-linked in ethyl acetate using a divinyl glycol cross linker and has a viscosity of between 4,000 and 11,000 centi-Poise measured at 25 °C. Information from the manufacturer indicates that Noveon® Polycarbophil AA-1 USP has the properties in the following table:

| Test | Specification | Lot Test Frequency¹ | Test Procedure² |
|---|---|---|---|
| Identification | | | |
| Colorimetric test | Pass | 1:200 | USP/NF |
| Gel formation test | Pass | 1:1 | USP/NF |
| Absorbing Power, g/g | 62 min | 1:1 | USP/NF |
| Viscosity, cP, 25°C | | | |
| Brookfield RVT, 20 rpm, neutralized to pH 7.3 - 7.8 | | | |
| 0.2 wt% mucilage, spindle #5 | 2,000-12,000 | 1:1 | Lubrizol 430-I³ |
| Loss on Drying, % | 1.5 max | 1:1 | USP/NF |
| pH, 1% Dispersion | 4.0 max | 1:1 | USP/NF |
| Residual Solvent⁴ | | | |
| Ethyl acetate, % | 0.45 max | 1:1 | Lubrizol SA-009 |
| Benzene, ppm | 0.50 max | 1:1 | Lubrizol SA-064 |
| Residual Monomer, ppm | | | |
| Free acrylic acid | 3,000 max | 1:1 | Lubrizol SA-005 |
| Sulphated Ash, % (Residue on ignition) | 4.0 max | 1:100 | USP/NF |

### The Melt Extrusion Process:

The Inventors have quite surprisingly found that the thin strip formulation of the present invention allows for treatment under mild process conditions (e.g. low temperature, shear, and pressure, *inter alia*) thereby preserving the thin strips components. The thin strip formulation can be melt extruded at melt temperatures below 150°C (e.g. below 100 °C, 90 °C, 80°C, 70 °C, 60 °C, and in some embodiments even below 50 °C, for example at 40 °C or 45 °C). Due to frictional stresses incurred within the melt extruder the melt temperature of components is often a few degrees more than the set point temperature of the extruder. Therefore, care should be taken to ensure the melt temperature of the components within the extruder is within these ranges. Furthermore, it is preferred to select an extruder screw speed and throughput flow rate where frictional temperature gains within the extruder are minimized. As described herein, frictional stresses upon the composition can also lead to degradation of the strip components and volatilization of the nicotine salt. Forming the thin strip formulation at these mild process conditions allows for preservation of components and preservation of the nicotine salt. The invention therefore also provides a method of forming a thin strip at these mild process conditions as well as thin strips formed at these mild conditions.

The hot melt extrusion composition of the present invention may be formed prior to introduction to the extruder or within the extruder itself. Where the composition is formed prior to introduction to the extruder it is preferred that the temperature profile of the extruder and subsequent processes (e.g. calendering) be maintained at a temperature of less than the melting point of the sugar alcohol or the sugar alcohol and the coating material of the API. Where the sugar alcohol is mannitol this temperature should not exceed 150 °C. Where the sugar alcohol is sorbitol this temperature should not exceed 90 °C. In most preferred embodiments this temperature will be between 40 and 80 °C (e.g. does not exceed 80 °C and more preferably does not exceed 70 °C, for example the temperature is between 50 and 70 °C) to prevent melting of the sugar alcohol and to prevent volatilization loss and degradation of the organic addition salt of nicotine.

Where the composition is formed within the extruder (e.g. by side stuffing one or more of the components) certain portions of the extruder may be operated at temperatures greater than those described above, thereby treating some of the components of the composition at elevated temperature for extended periods of time. In the later embodiment it is again preferred to minimize exposure of the nicotine salt to elevated temperature for an extended period of time. Therefore, in another preferred embodiment the organic addition salt of nicotine is introduced/side-stuffed to the extruder in a downstream barrel section from where other components are introduced. For example a portion or all of the organic addition salt of nicotine is side-stuffed into the extruder and the extrusion composition is formed and thoroughly mixed by the time the barrel exit section (e.g. the die) of the extruder is reached. The upstream barrel sections from the organic addition salt of nicotine side stuffing barrel(s) may be operated at elevated temperatures. The side-stuffing barrel section and downstream barrel sections are preferably operated under the preferred temperatures ranges described above.

Upon exit from the extruder the extrudate may be calendered to its desired thickness using one or more optionally temperature-controlled calendering rolls. Where the rolls are temperature controlled, it is preferred to select a temperature where the extrudate does not stick to the rolls. The controlled roll temperature can be for example between 10 °C and 100 °C, more preferably between 20 and 70 °C, for example between 25 and 50 °C (e.g. 30 °C). In preferred embodiments the thickness of the thin strip is between 0.05 mm and 2 mm, for example between 0.1 mm and 0.8 mm (e.g. between 0.2 mm and 0.5 mm). In other preferred embodiments the thickness of the thin strip is less than 0.4 mm, for example 0.3 mm or 0.25 mm.

The calendered composition can then be introduced to a backing material and then rolled to form a master roll. The master roll then can be cut into feeder rolls having the desired thin strip width or length and then unwound and cut or scribed to form dosage size thin strips. The amount of the organic addition salt of nicotine in the thin strip will be a function of the size of the thin strip (length x width x thickness) and the concentration of the organic addition salt of nicotine in the composition. In a preferred embodiment, an individual thin strip will contain a recommended dose of the organic addition salt of nicotine. In preferred embodiments, the thin strip will be from 0.5 to 4 cm wide by 0.5 to 6 cm long. In other embodiments the thin strip will be from 1.5 to 3 cm wide (e.g. about 2 cm wide) by 1.5 to 5 cm long (e.g. about 3.5 cm long). In another embodiment, the thin strips preferably have dimensions of 22 mm x 16 mm to 22 mm x 37 mm. In another embodiment the test strip is formed such that the total surface area of the strip is between 1 cm² to 20 cm² (e.g. between 1 cm² and 10 cm²). Once in individual thin strip form the strips may be individually packaged or combined with others and packed in a multiple dose container (e.g. in ribbon/dispenser for stacked form). In another embodiment the strip is cut so that it has between 1 and 8 mg of nicotine (free base equivalent), for example 2 or 4 mg of nicotine.

As described herein, the formulation and techniques of the present invention allow for the preparation of thin strips compositions containing an organic addition salt of nicotine. The processes described allow for the preservation of the organic addition salt of nicotine. In most preferred embodiment the thin strip formation methods of the present invention will produce a thin strip that contains greater than 80 wt%, 90wt%, 95 wt%, 97 wt%, 99 wt%, or more of the organic addition salt of nicotine present in the initial melt extrusion composition.

### EXAMPLES:

Having described the invention in detail, the following examples are provided. The following examples provide acceptable and preferred strategies of forming test strips that are acceptable for use in industry.

### 1 Extrusion of placebo films and thermal characterization of nicotine bitartrate by TGA

### 1.1 Introduction

PCT Patent application PCT/US09/69793 entitled "MELT EXTRUDED THIN STRIPS CONTAINING COATED PHARMACEUTICAL" filed on December 30, 2009, discloses a hot melt extrusion composition containing high molecular weight PEO, PEG, a sugar alcohol, and coated API granules. In that reference, it was unexpectedly found that the composition could be melt extruded under mild conditions (e.g. low temperature) to form thin strips having a quick dissolution time and other desirable properties.

Due to the ability of this composition to be melt extrudable at these mild processing conditions, the base components of PEO, PEG, and sugar alcohol were selected for the initial composition of melt extruded films containing the volatile and temperature sensitive organic addition salt of nicotine (e.g. bitartrate dihydrate).

To test the feasibility of formulating nicotine bitartrate dihydrate into melt extruded films having prodesirable properties, initial studies were performed. Placebo film formulations were investigated to select a polymer to prolong the film disintegration time.

Nicotine bitartrate dihydrate has a low melting point, and the free base, nicotine, is known to be volatile and flammable. Thermal gravimetric analysis (TGA) was used to study the response of the nicotine salt to elevated temperatures.

### 1.2 Placebo Film Preparation

To extend film cohesion, higher molecular weight polymers were added to a base formulation containing PEO, PEG, and mannitol. The higher weight polymers also impart bioadhesive properties to the film. Polyox Coagulant was chosen since a lower molecular weight grade of the same polymer (PEO), Polyox WSR N10 (MW 100k) was already part of the formulation, and any issues arising from insufficient polymer miscibility were avoided. Polycarbophil and Carbomer 974P are known to be excellent bioadhesive polymers. They are high molecular weight polymers of acrylic acid, chemically crosslinked with divinyl glycol and allyl ethers of pentaerythritol, respectively. Table 1 lists the placebo films melt extruded for this study, and Table 2 shows the processing conditions for the films.

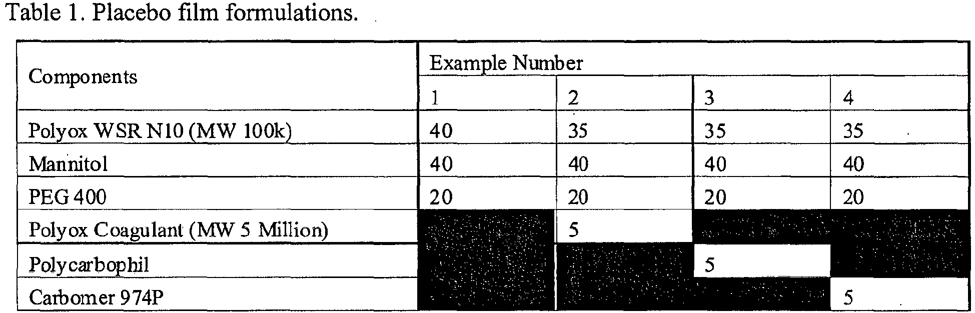

**Table 2. Processing conditions for melt extruded films.**

| Processing conditions | Example Number | | | |
|---|---|---|---|---|
| | 1 | 2 | 3 | 4 |
| Extrusion temperature, °C | 55 | 55 | 55 | 55 |
| Feed rate, kg/hr | 1.0 | 1.0 | 1.0 | 1.0 |
| Screw speed, RPM | 50 | 125 | 125 | 125 |

### 1.2.1 Placebo Films - Disintegration testing

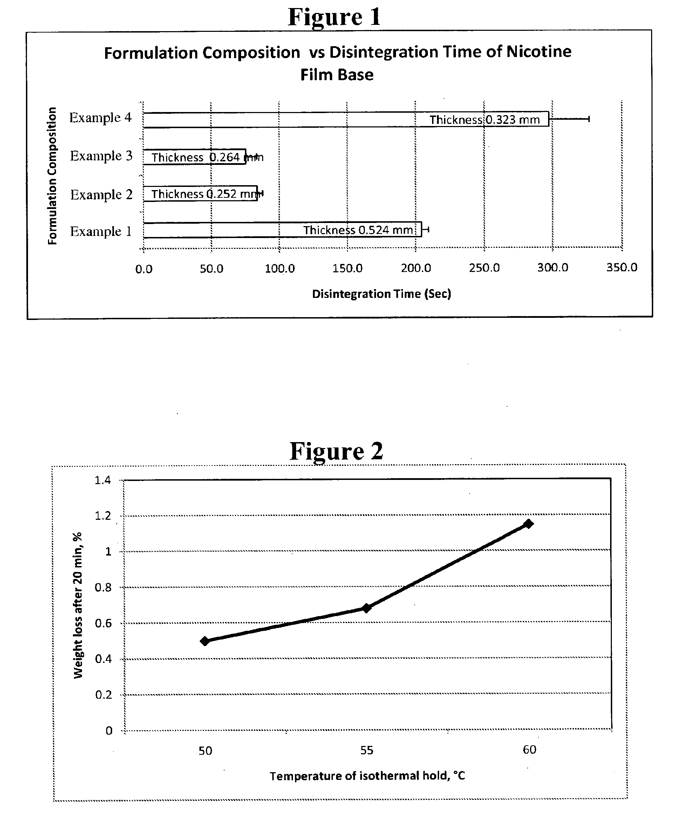

Film samples (about 2 cm x 0.65 cm) were punched from films using a stainless steel punch and a mallet. Samples were clipped into sinkers (small paperclips, n=3), and were tested in a USP disintegration tester in de-ionized water (36.5 °C). Average disintegration times are shown in Figure 1. Thickness affected disintegration time, as expected.

Figure 1 indicates that film of Example 4 had the longest disintegration time, and the highest ratio of disintegration time and film thickness.

### 1.3 Thermo-gravimetric analysis of Nicotine bitartrate dihydrate

During thermo-gravimetric analysis (TGA), the weight of a sample is measured very accurately as the sample is undergoing a temperature program (usually a gradual increase in temperature). Changes in the sample that result in weight differences will be detected, and can be correlated to temperature. This technique was used to investigate the onset temperatures of dehydration and degradation of the nicotine salt.

### 1.3.1 Temperature scan 30°C to 950°C

Table 4 details the temperature program for this study. The sample was heated from 30 to 950°C under nitrogen (inert gas) or air.

Two events were visible in TGA thermograms: The first one resulted in about 4% weight loss; its onset is at 73°C and inflection at 82°C. This event is probably due to loss of water dihydrate structure. The second event has an onset point of 191 °C and an inflection at 203°C. This was considered to be due to thermal decomposition of the API. Literature suggests that the weight loss occurs as Nicotine bitartrate is converted to Nicotine free base, which is volatile (Jeffrey I. Seeman, Jay A Fournier, John B. Paine III, and Bruce E. Waymack. The Form of Nicotine in Tobacco. Thermal Transfer of Nicotine and Nicotine Acid Salts to Nicotine in the Gas Phase. J. Agric. Food Chem., 1999, 47 (12), pp 5133-5145). Regardless of the reason for the weight loss, the onset temperature of this event presents the upper limit if the processing temperature window.

The API is known to be sensitive to oxidation, and to investigate the effect of oxygen on possible degradation, samples were run separately under air as well as under nitrogen. The onset temperatures were not affected by the gas type (Table 5), which can be interpreted as supporting the view that nicotine escaped into the gas phase rather than decomposed.

The extrusion temperatures are lower than the onset temperatures for the thermal events detected by TGA, underlining the importance of the processing conditions. These results also indicate that TGA analysis under air did not accelerate weight loss.

**Table 3. Profile of the TGA temperature program.**

| Event | Temperature (°C) | To Temperature (°C) | Rate (°C/min) | Time (min) |
|---|---|---|---|---|
| Hold | 30 | - | - | 2 |
| Heat | 30 | 400 | 10 | - |
| Heat | 400 | 950 | 50 | - |
| Hold | 950 | - | - | 3 |
| Cool | 950 | 30 | 50 | - |
| Hold | 30 | - | - | 1 |

**Table 4. Onset temperatures of thermal events**

| Run | First Event Onset Temperature (°C) | Second Event Onset Temperature (°C) |
|---|---|---|
| Nitrogen purged sample | 72.54 | 190.78 |
| Air purged sample | 73.93 | 190.23 |

### 1.3.2 Isothermal hold

Irrespective of events at higher temperatures, tests were conducted to determine how Nicotine bitartrate dihydrate was affected by the extrusion processing temperatures for the duration of the process. The sample weight was monitored for 20 minutes at probable extrusion temperatures of 50, 55 and 60°C. This duration is longer than expected process time for small batches including hold time in hoppers.

After a 20 minute isothermal hold, weight loss of nicotine bitartrate dihydrate was 0.497%, 0.678% and 1.147% for 50, 55 and 60°C, respectively (Figure 2: Effect of isothermal hold on weight of Nicotine bitartrate dihydrate). The nature of the event is probably related to the loss of molecular hydration water, as the main weight loss in TGA occurred at much higher temperatures. The extent of the weight loss in this study is shown to be related to the hold temperature. These results indicate that the extrusion temperature should be minimized.

### 1.4 Summary

A placebo formulation containing 5% Carbomer 974P was chosen as the preliminary composition for films containing Nicotine bitartrate based on unexpected film disintegration values. However, disintegrating films containing polycarbophil were also shown to be prepared at low extrusion temperatures and mild processing conditions.

TGA analysis of nicotine bitartrate dihydrate showed two weight loss events, both above the current extrusion temperatures. The temperatures at which weight loss occurred were not affected by the analysis gas (air or nitrogen). Some temperature-depended weight loss was observed under isothermal holds for 20 minutes in the processing temperature range.

### 2 Initial extrusion of thin films containing nicotine bitartrate dihydrate

### 2.1 Introduction

After choosing the preliminary formulation, the purpose of the initial extrusion of films containing Nicotine bitartrate was to determine if the active could be melt extruded without significant losses due to the extrusion process. In addition, two alkaline salts were added to the formulation to investigate if the presence of solid salts during extrusion affected the stability of Nicotine bitartrate. To this end, films were prepared under different processing conditions, and analyzed for potency and film properties.

### 2.2 Film Compositions

Carbopol 974P (5%) was added as a bioadhesive agent, and to extend the disintegration time of the film. A basic pH is desirable to facilitate nicotine absorption in the mouth by liberating the free base form of Nicotine bitartrate. Two salts were chosen as buffering agents that impart an alkaline pH, magnesium oxide, which is poorly soluble, and potassium phosphate monobasic, which is highly soluble. The film compositions are listed in Table 5.

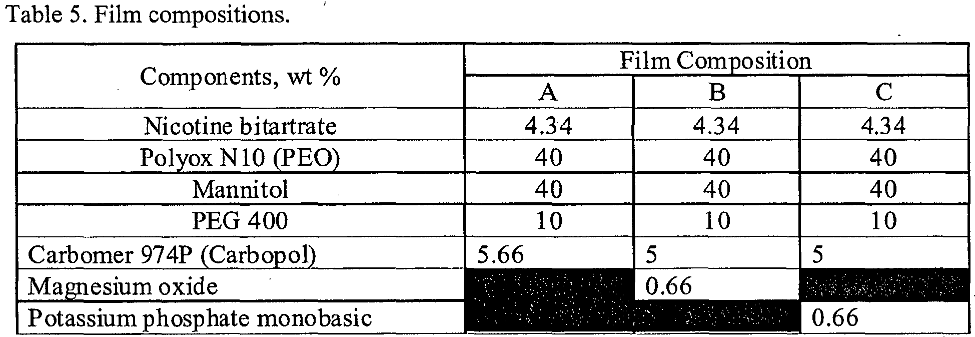

### 2.3 Melt extrusion of films

The objective in the present study was to explore the behavior of Nicotine bitartrate during melt extrusion, the basic film blend containing the API (comp. A) was extruded over the entire temperature range, while the salt-containing films were extruded only at the lowest temperature (Table 6). Films were successfully prepared at all extrusion temperatures.

**Table 6. Processing Conditions.**

| Example Number | Extrusion temperature, °C | Screw Speed, RPM |
|---|---|---|
| 5 (Comp A) | 55 | 125 |
| 6 (Comp A) | 75 | 125 |
| 7 (Comp A) | 95 | 125 |
| 8 (Comp B) | 55 | 125 |
| 9 (Comp C) | 55 | 125 |

### 2.4 Characterization of melt extruded films containing Nicotine bitartrate dihydrate

The objective of the study was to investigate if nicotine bitartrate dihydrate can be melt extruded, and to measure the loss of API during the process. In addition, film characteristics were investigated.

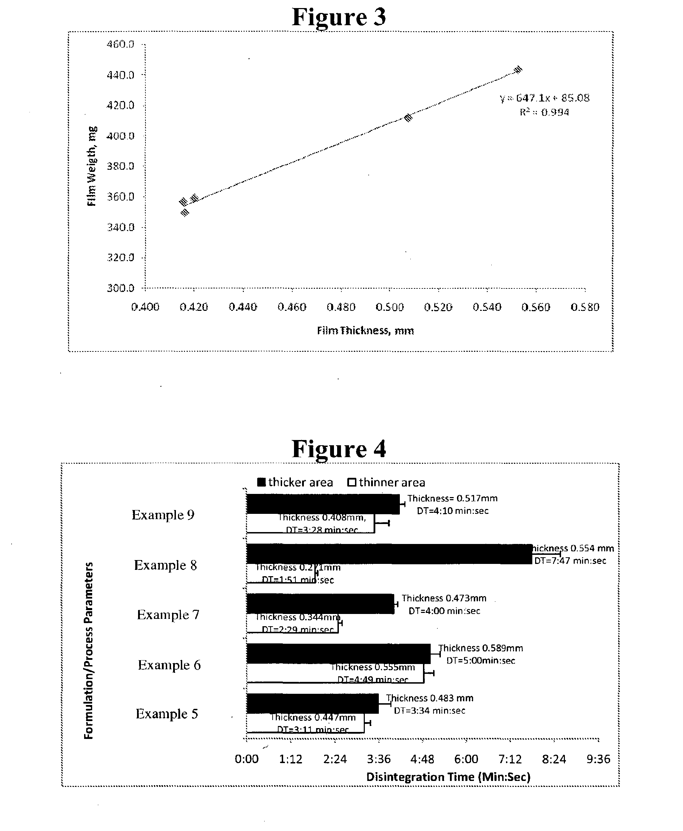

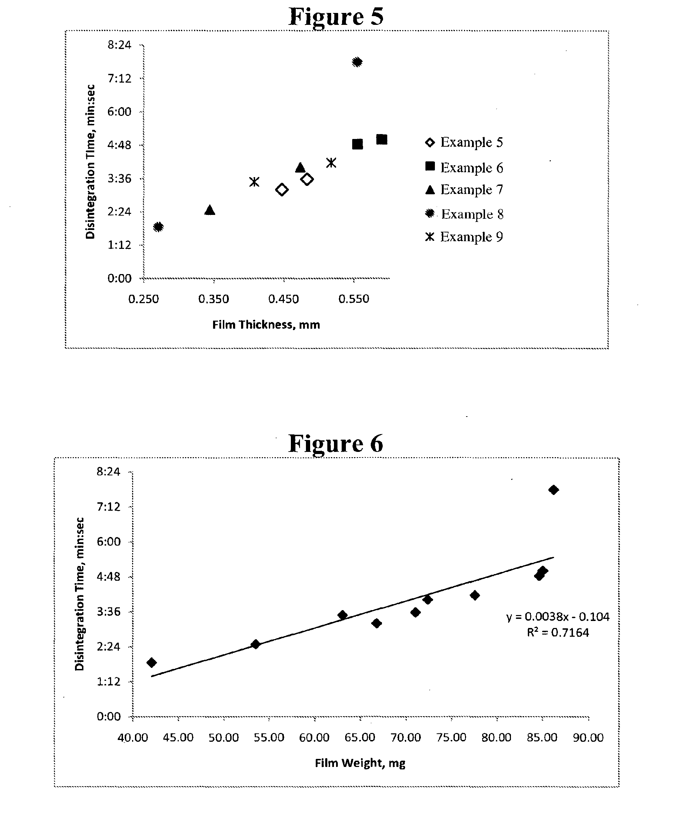

Film thickness and film weight showed a linear correlation (R2=0.994, Figure 3), as expected, since the formulation was similar to films containing Dextromethorphan HBr, and those films were shown to have similar correlations. The disintegration times (Figure 4) were affected by film thickness and film weight. When data from all formulations was used, a linear correlation of film thickness and disintegration times (R2=0.646, Figure 5, trend line not shown) as well as film weight and disintegration times (R2=0.716, Figure 6), was found.

In Figure 5 and Figure 6, the outlaying value in the upper right quadrant of the graph was due to the heaviest and thickest film sample, originating from Example 8, which disintegrated in 7:47 min. Since its sample thickness and weight were nonetheless similar to that of two faster-disintegrating films, which disintegrated in the 4-5 minute range, it is likely that the film composition contributed to the longer disintegration time. These results indicate that the low solubility of the added pH regulator salt was responsible for the extended disintegration time of thick films, since an equivalent formulation with a readily dissolving salt (potassium phosphate monobasic, Example 9), had a lower disintegration time at similar film thickness.

### 2.5 Characterization of the drug content of films containing nicotine bitartrate dihydrate

### 2.5.1 Drug Content and potency

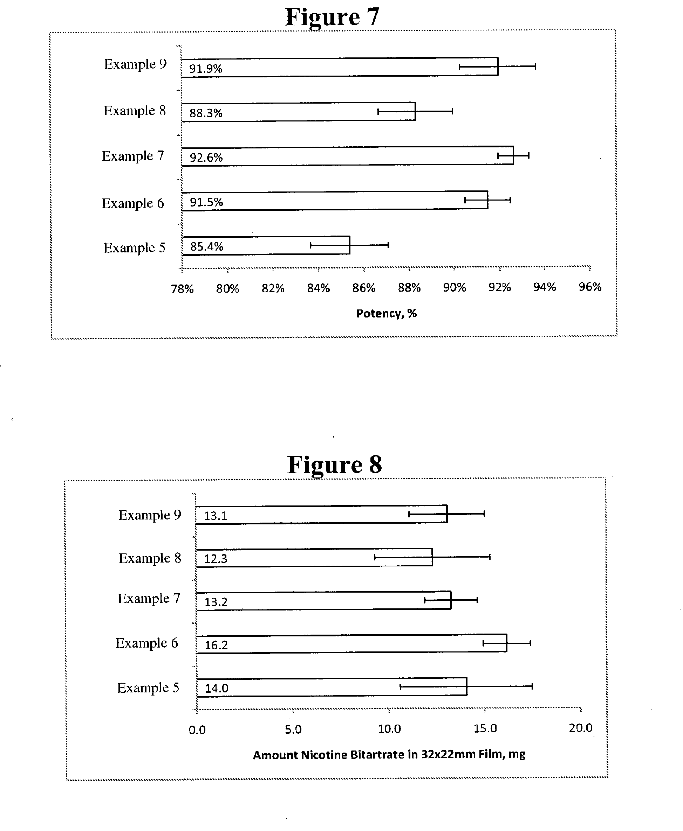

Original potency results of melt extruded films containing nicotine bitartrate ranged from 85.4% to 92.6%, adjusted for API purity and hydration water content (Figure 7). It was suspected that the low purity was related to the extraction method, and was not due to an effect of melt extrusion on the API. After optimization of the extraction method and HPLC method, potency of Example 9 increased from 92.0 ± 1.6% to 96.6 ± 1.1%. The other films were not been tested with the improved analytical method.

Figure 7 shows the potency of melt extruded films containing Nicotine bitartrate dihydrate as determined by the original method.

An initial test of the method's efficiency to find Nicotine bitartrate in a sample was the analysis of a powder blend by the same extraction process as the film. The blend was prepared on an analytical balance to have the same composition as films of examle 5-7. Nicotine bitartrate dihydrate potency was found to be 88.1%±0.01 (SD, n=6), which confirmed that the extraction method, rather than the process, influenced the results.

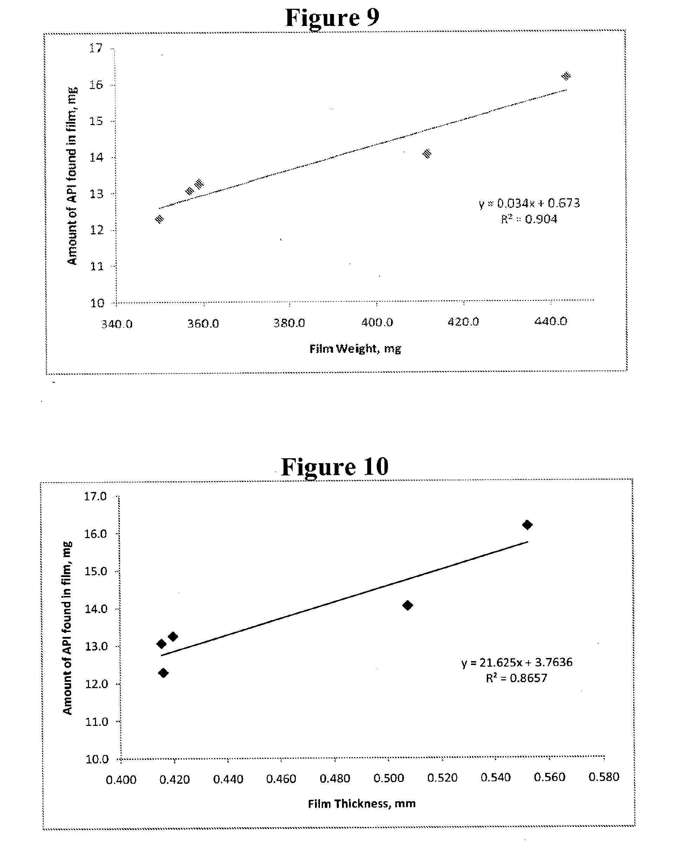

The total amount of API per film strip dose is an important consideration for further development. Even with the inefficient extraction process, a 32x22mm film contained at least between 12.3 and 16.2 mg Nicotine bitartrate dihydrate (Figure 8). This was above the targeted dose for the salt (6.14 mg), and thus opened opportunities to demonstrated smaller film sizes could be made that contain a desired dose of the API. The amount of API in the film correlated with the film weight (R2=0.904, Figure 9), and somewhat less with the film thickness (R2=0.865, Figure 10).

### 2.6 Thermal properties of films

To understand the state of the API in the film, differential scanning calorimetry (DSC) was used to analyze thermal properties of the films. Films without added buffers (Example 5), with an insoluble salt (magnesium oxide, Example 8) and with a soluble salt (potassium phosphate monobasic, Example 9) were analyzed. Nicotine bitartrate dihydrate powder was investigated as a control.

Samples (about 15 mg film or about 2 mg API powder, n=2) were accurately weighed into 30 microliter aluminum pan. The sealed pan was subjected to the following temperature program:
1. Hold at -50°C for 1 minute.
2. Heat from -50°C to 130°C at 20°C/min.

The higher limit of the program was chosen to be 130 °C to scan far enough past the melting point of the pure API (about 91°C), and yet to avoid proximity of the onset of degradation as detected by TGA (about 190°C). The scan rate, 20 °C/min, was considered a compromise between fast scanning and accuracy of event temperatures.

Thermograms for all films were similar, and showed two events: a large endothermic peak at 58-59 °C, and a smaller endothermic peak between 85-87 °C. Data for all formulation was in the same range (Table 7). The first peak was ascribed to the melting of PEO, and the second peak was considered to be the melting point of the nicotine salt. The analysis of the nicotine salt showed a single event, an endothermic peak at 91-92 °C. Thus the melting point of the API in the film was depressed compared to the pure substance, which was not surprising considering that the salt was in mixture. Of the other film components, PEG 400 is liquid, without a thermal event in the temperature range, and Mannitol has a melting point of 167 °C, which was outside of the temperature range of the analysis.

The thermal data leads to the conclusion that the addition of a small amount of buffer salt (0.66 wt %) to the formulation has no effect on the state of the film as detected by DSC. If the alkaline buffer had resulted in a transformation of the nicotine salt to the nicotine free base, the extent of the thermal event associated with the melting of the salt would be affected, since the base is oily at room temperature, and would not exhibit an endotherm at 85-87 °C.

**Table 7. Thermal characteristics of melt extruded films containing Nicotine bitartrate.**

| Sample | Thermal Event Nr. 1, °C | | Thermal Event Nr. 2, °C | |
|---|---|---|---|---|
| | Sample 1 | Sample 2 | Sample 1 | Sample 2 |
| 5 | 58.0 | 58.6 | 85.0 | 86.6 |
| 8 | 59.5 | 59.5 | 87.1 | 87.0 |
| 9 | 58.5 | 58.7 | 87.5 | 87.0 |
| Nicotine Bitartrate | N/A | N/A | 91.5 | 92.2 |

### 2.7 Summary

Films containing nicotine bitartrate dihydrate were extruded at 55, 75 and 95°C, and yielded films of satisfactorily texture and appearance. Thickness and weight of the films were closely correlated (R2=0.994), and the disintegration time showed a looser correlation to both film thickness (R2=0.646) and film weight (R2=0.716). Potency was determined in films after extraction of the API, and ranged from 85.4 to 92.6%, which corresponded in 32x22mm films to 12.3 to 16.2 mg/film sample. The dose of API per film correlated with film weight (R2=0.904) and film thickness (R2=0.865). The extraction method used in this study was 100% efficient in extracting the API, as extraction from the powder blend recovered only 88.1 %±0.01 (SD, n=6) of the drug. The addition of 0.66 wt% alkaline buffer salt (either magnesium oxide or potassium phosphate monobasic) had no effect on the melting peak of the nicotine salt as detected by DSC, though the melting point was depressed from the pure material (85-87 °C and 91-92 °C, respectively).

## Claims

1. A melt extruded thin strip comprising:
10 to 80 % by weight of polyethylene oxide having a molecular weight of from 70,000 to 300,000 daltons;
5 to 50 % by weight of a sugar alcohol having a melting point in excess of 75°C;
5 to 30 % by weight of polyethylene glycol having a molecular weight of from 100 to 4,000 daltons;
1 to 30 % by weight of a carboxy vinyl polymer cross linked with an allyl ether of pentaerythritol; and
1 to 10 % by weight of an organic acid addition salt of nicotine, wherein the thin strip is between 0.05 millimeters and 2.00 millimeters thick,
wherein the sugar alcohol is selected such that its melting point is in excess of the highest temperature at which the formulation will be treated during formation of the thin strip.

2. The thin strip of claim 1, wherein the sugar alcohol comprises mannitol or sorbitol.

3. The thin strip of claim 1, wherein the thin strip comprises:
25 to 55 % by weight of polyethylene oxide having a molecular weight of from 100,000 to 200,000 daltons;
35 to 45 % by weight of a sugar alcohol having a melting point in excess of 150°C;
7 to 15 % by weight of polyethylene glycol having a molecular weight of from 300 to 600 daltons;
5 to 15 % by weight of a carboxy vinyl polymer cross linked with an allyl ether of pentaerythritol; and
2 to 6 % by weight of an organic acid addition salt of nicotine.

4. The thin strip of any of claims 1 to 3, further comprising an alkaline buffer selected from the group consisting of potassium phosphate monobasic, sodium carbonate, sodium bicarbonate, sodium chloride, sodium phosphate dibasic and sodium phosphate monobasic.

5. The thin strip of any of claims 1 to 4, wherein the carboxy vinyl polymer cross linked with an allyl ether of pentaerythritol is a polymer that is swellable in water.

6. The thin strip of any of claims 1 to 5, wherein the carboxy vinyl polymer cross linked with an allyl ether of pentaerythritol has a glass transition temperature of between 100 °C and 110 °C in powder form.

7. A method of forming a thin strip according to any of claims 1 to 6, the method comprising the steps of:
(I) forming a composition comprising thin strip components of the polyethylene oxide, the sugar alcohol, the polyethylene glycol, the carboxy vinyl polymer, the organic acid addition salt of nicotine, and optionally the alkaline buffer of claim 4,
(II) melt extruding a thin sheet having to a thickness of between 0.05 millimeters and 2.00 millimeters from the composition; and
(III) cutting the thin sheet into thin strips;
wherein the processing temperature during steps (I), (II), and (III) does not exceed the melting point temperature of the sugar alcohol.

8. The method of claim 7, wherein the sugar alcohol comprises mannitol and the melt temperature during steps (I), (II), and (III) does not exceed 150°C.

9. The method of claims 7 or 8, wherein the sugar alcohol comprises sorbitol and the melt temperature during steps (I), (II), and (III) does not exceed 90°C.

10. The method of any of claims 7 to 9, wherein the melt temperature during steps (I), (II), and (III) does not exceed 80°C.

11. The method of any of claims 7 to 10, wherein the melt temperature during steps (I), (II), and (III) does not exceed 70°C.

12. The method of any of claims 7 to 11, wherein the thin strip is 0.1 to 0.8 millimeters thick.

13. The method of any of claims 7 to 12, wherein the composition is formed in an extruder during hot melt extrusion step (II), wherein a portion of the organic acid addition salt of nicotine is introduced to the extruder in a downstream barrel from where other thin strip components are introduced.

14. The method of any of claims 7 to 13, wherein steps (I), (II), and (III) are performed such that the thin strip has 2 mg or 4 mg of nicotine (free base equivalent).

15. The method of any of claims 7 to 14, wherein the melt extruding step (II) further includes calendering the extrudate to the thickness of between 0.05 millimeters and 2.00 millimeters, or 0.1 to 0.8 millimeters.

## Patentansprüche

1. Schmelzextrudierter dünner Streifen, umfassend:
10 bis 80 Gewichts-% Polyethylenoxid mit einem Molekulargewicht von 70.000 bis 300.000 Dalton;
5 bis 50 Gewichts-% eines Zuckeralkohols mit einem Schmelzpunkt höher als 75°C;
5 bis 30 Gewichts-% Polyethylenglycol mit einem Molekulargewicht von 100 bis 4.000 Dalton;
1 bis 30 Gewichts-% eines Carboxyvinylpolymers, das mit einem Allylether von Pentaerythritol vernetzt ist; und
1 bis 10 Gwichts-% eines organischen Säureadditionssalzes von Nikotin, wobei der dünne Streifen zwischen 0,05 Millimeter und 2,00 Millimeter dick ist,
wobei der Zuckeralkohol so ausgewählt wird, dass sein Schmelzpunkt höher ist als die höchste Temperatur, bei der die Formulierung, während der Bildung des dünnen Streifens, behandelt wird.

2. Dünner Streifen nach Anspruch 1, wobei der Zuckeralkohol Mannitol oder Sorbitol umfasst.

3. Dünner Streifen nach Anspruch 1, wobei der dünne Streifen umfasst:
25 bis 55 Gewichts-% Polyethylenoxid mit einem Molekulargewicht von 100.000 bis 200.000 Dalton;
35 bis 45 Gewichts-% eines Zuckeralkohols mit einem Schmelzpunkt höher als 150°C;
7 bis 15 Gewichts-% Polyethylenglycol mit einem Molekulargewicht von 300 bis 600 Dalton;
5 bis 15 Gewichts-% eines Carboxyvinylpolymers, das mit einem Allylether von Pentaerythritol vernetzt ist; und
2 bis 6 Gwichts-% eines organischen Säureadditionssalzes von Nikotin.

4. Dünner Streifen nach einem der Ansprüche 1 bis 3, weiter umfassend einen alkalischen Puffer, ausgewählt aus der Gruppe bestehend aus Kaliumphosphat monobasisch, Natriumcarbonat, Natriumbicarbonat, Natriumchlorid, Natriumphosphat dibasisch und Natriumphosphat monobasisch.

5. Dünner Streifen nach einem der Ansprüche 1 bis 4, wobei das Carboxyvinylpolymer, das mit einem Allylether von Pentaerythritol vernetzt ist, ein Polymer ist, das quellbar in Wasser ist.

6. Dünner Streifen nach einem der Ansprüche 1 bis 5, wobei das Carboxyvinylpolymer, das mit einem Allylether von Pentaerythritol vernetzt ist, eine Glasübergangstemperatur zwischen 100°C und 110°C in Pulverform aufweist.

7. Verfahren zum Bilden eines dünnen Streifens nach einem der Ansprüche 1 bis 6, wobei das Verfahren die Schritte umfasst von:
(I) Bilden einer Zusammensetzung, umfassend dünner Streifen-Komponenten des Polyethylenoxids, des Zuckeralkohols, des Polyethylenglycols, des Carboxyvinylpolymers, des organischen Säureadditionssalzes von Nikotin und gegebenenfalls des alkalischen Puffers von Anspruch 4,
(II) Schmelzextrudieren einer dünnen Platte mit einer Dicke zwischen 0,05 Millimeter und 2,00 Millimeter aus der Zusammensetzung; und
(III) Schneiden der dünnen Platte in dünne Streifen;
wobei die Herstellungstemperatur während Schritten (I), (II) und (III) die Schmelzpunkt-Temperatur des Zuckeralkohols nicht übersteigt.

8. Verfahren nach Anspruch 7, wobei der Zuckeralkohol Mannitol umfasst und die Schmelztemperatur während Schritten (I), (II) und (III) 150°C nicht überschreitet.

9. Verfahren nach Anspruch 7 oder 8, wobei der Zuckeralkohol Sorbitol umfasst und die Schmelztemperatur während Schritten (I), (II) und (III) 90°C nicht überschreitet.

10. Verfahren nach einem der Ansprüche 7 bis 9, wobei die Schmelztemperatur während Schritten (I), (II) und (III) 80°C nicht überschreitet.

11. Verfahren nach einem der Ansprüche 7 bis 10, wobei die Schmelztemperatur während Schritten (I), (II) und (III) 70°C nicht überschreitet.

12. Verfahren nach einem der Ansprüche 7 bis 11, wobei der dünne Streifen 0,1 bis 0,8 Millimeter dick ist.

13. Verfahren nach einem der Ansprüche 7 bis 12, wobei die Zusammensetzung in einem Extruder während Heiße-Schmelze-Extrusionsschritt (II) gebildet wird, wobei ein Teil des organischen Säureadditionssalzes von Nikotin zu dem Extruder in einem stromabwärtigen Rohr, woher andere dünne Streifenkomponenten zugeführt werden, zugeführt wird.

14. Verfahren nach einem der Ansprüche 7 bis 13, wobei Schritte (I), (II) und (III) durchgeführt werden, so dass der dünne Streifen 2 mg oder 4 mg Nikotin (freie Baseäquivalente) aufweist.

15. Verfahren nach einem der Ansprüche 7 bis 14, wobei der Schmelzextrusionsschritt (II) weiter Kalandrieren des Extrudats auf die Dicke zwischen 0,5 Millimeter und 2,00 Millimeter oder 0,1 bis 0,8 Millimeter umfasst.

## Revendications

1. Bande mince extrudée à l'état fondu comprenant :
10 à 80 % en poids d'oxyde de polyéthylène ayant une masse moléculaire allant de 70 000 à 300 000 daltons ;
5 à 50 % en poids d'un alcool glucidique ayant un point de fusion supérieur à 75 °C ;
5 à 30 % en poids de polyéthylène glycol ayant une masse moléculaire de 100 à 4 000 daltons ;
1 à 30 % en poids d'un polymère de vinyle carboxy réticulé avec un éther allylique de pentaérythritol ; et
1 à 10 % en poids d'un sel d'addition d'acide organique de nicotine, dans lequel la bande mince est entre 0,05 millimètre et 2,00 millimètres d'épaisseur,
dans lequel l'alcool glucidique est sélectionné de sorte que son point de fusion est supérieur à la température la plus élevée à laquelle la formulation sera traitée pendant la formation de la bande mince.

2. Bande mince selon la revendication 1, dans laquelle l'alcool glucidique comprend du mannitol ou du sorbitol.

3. Bande mince selon la revendication 1, dans laquelle la bande mince comprend :
25 à 55 % en poids d'oxyde de polyéthylène ayant une masse moléculaire allant de 100 000 à 200 000 daltons ;
35 à 45 % en poids d'un alcool glucidique ayant un point de fusion supérieur à 150 °C ;
7 à 15 % en poids de polyéthylène glycol ayant une masse moléculaire de 300 à 600 daltons ;
5 à 15 % en poids d'un polymère de vinyle carboxy réticulé avec un éther allylique de pentaérythritol ; et
2 à 6 % en poids d'un sel d'addition d'acide organique de nicotine.

4. Bande mince selon l'une quelconque des revendications 1 à 3, comprenant en outre un tampon alcalin sélectionné à partir du groupe constitué par du phosphate de potassium monobasique, du carbonate de sodium, du bicarbonate de sodium, du chlorure de sodium, du phosphate de sodium dibasique et du phosphate de sodium monobasique.

5. Bande mince selon l'une quelconque des revendications 1 à 4, dans laquelle le polymère de vinyle carboxy réticulé avec un éther allylique de pentaérythritol est un polymère qui est susceptible de gonfler dans l'eau.

6. Bande mince selon l'une quelconque des revendications 1 à 5, dans laquelle le polymère de vinyle carboxy réticulé avec un éther allylique de pentaérythritol a une température de transition vitreuse entre 100 °C et 110 °C sous forme de poudre.

7. Procédé de formation d'une bande mince selon l'une quelconque des revendications 1 à 6, le procédé comprenant les étapes de :
(I) formation d'une composition comprenant des composants de bande mince de l'oxyde de polyéthylène, de l'alcool glucidique, du polyéthylène glycol, du polymère de vinyle carboxy, du sel d'addition d'acide organique de nicotine, et de manière facultative du tampon alcalin de la revendication 4,
(II) extrusion à l'état fondu d'une feuille mince à une épaisseur entre 0,05 millimètre et 2,00 millimètres à partir de la composition ; et
(III) découpe de la feuille mince en bandes minces ;
dans lequel la température de traitement pendant des étapes (I), (II) et (III) ne dépasse pas la température de point de fusion de l'alcool glucidique.

8. Procédé selon la revendication 7, dans lequel l'alcool glucidique comprend du mannitol et la température de fusion pendant les étapes (I), (II) et (III) ne dépasse pas 150 °C.

9. Procédé selon les revendications 7 ou 8, dans lequel l'alcool glucidique comprend du sorbitol et la température de fusion pendant les étapes (I), (II) et (III) ne dépasse pas 90 °C.

10. Procédé selon l'une quelconque des revendications 7 à 9, dans lequel la température de fusion pendant les étapes (I), (II) et (III) ne dépasse pas 80 °C.

11. Procédé selon l'une quelconque des revendications 7 à 10, dans lequel la température de fusion pendant les étapes (I), (II) et (III) ne dépasse pas 70 °C.

12. Procédé selon l'une quelconque des revendications 7 à 11, dans lequel la bande mince fait 0,1 à 0,8 millimètre d'épaisseur.

13. Procédé selon l'une quelconque des revendications 7 à 12, dans lequel la composition est formée dans une extrudeuse pendant une étape d'extrusion à l'état fondu à chaud (II), dans lequel une partie du sel d'addition d'acide organique de nicotine est introduite dans l'extrudeuse dans un baril aval à partir duquel d'autres composants de bande mince sont introduits.

14. Procédé selon l'une quelconque des revendications 7 à 13, dans lequel les étapes (I), (II) et (III) sont effectuées de sorte que la bande mince a 2 mg ou 4 mg de nicotine (équivalent de base libre).

15. Procédé selon l'une quelconque des revendications 7 à 14, dans lequel l'étape d'extrusion à l'état fondu (II) inclut en outre le calandrage de l'extrudat à l'épaisseur entre 0,05 millimètre et 2,00 millimètres, ou 0,1 à 0,8 millimètre.
